# EUROPEAN PATENT SPECIFICATION

(11) **EP 2 256 651 B1**
(45) Date of publication and mention of the grant of the patent: **12.09.2018**
(21) Application number: 10004781.0
(22) Date of filing: 06.05.2010
(51) Int. Cl.: G06F 19/00

(54) **Control system for magnetic resonance scanner**
Steuerungssystem für ein Magnetresonanzscanner
Système de commande pour un scanner à résonance magnétique

(30) Priority: 27.05.2009 IT MI20090935
(43) Date of publication of application: 01.12.2010
(73) Proprietor: Stagnaro, Riccardo, 16040 San Colombano Certenoli (GE) (IT)
(72) Inventor: Stagnaro, Riccardo, 16040 San Colombano Certenoli (GE) (IT)
(74) Representative: Lunati, Valerio

(56) References cited:
- WO-A1-03/098245
- US-A- 6 157 194
- US-A1- 2004 267 117
- US-A1- 2005 063 575
- US-A1- 2006 235 671
- US-A1- 2009 040 565

## Description

The present invention relates to a control system for a magnetic resonance scanner of the type pointed out in the preamble of the first claim.

Presently known are scanners for magnetic resonance images or sectional radiographies or tomographies, merely referred to as MRI images.

They are governed by suitable electronic systems adapted to set the scanner itself and to regulate the magnets thereof and the different coils present inside said scanner.

A similar system is described in patent application US-A-2005/063575.

They have some important drawbacks.

In particular regulation of the different sequences of action of the magnets and the coils being part of the scanner itself appears to be very complicated.

In addition, also complicated is use of said scanners by the responsible staff, such as in particular sequence developers and physicians, as well as data acquisition following the action of said magnets and coils present in the scanner.

Under this situation, the technical task underlying the present invention is to devise a control system for a magnetic resonance scanner capable of substantially obviating the mentioned drawbacks.

Within the scope of this technical task it is an important aim of the invention to obtain a control system for a magnetic resonance scanner allowing a simple regulation of the different sequences of action of the magnets and coils being part of the scanner itself while at the same time obtaining a system capable of implementing and executing complex examinations or tests such as spin-echo, fast spin-echo, gradient-echo also with multiple echoes and high number of slices and, when necessary, supporting reduced execution times (sequences with fast acquisitions), further enabling the necessary architectural flexibility to be available for adapting itself to the development and experimentation of new MRI sequences implemented for research purposes and for developing new examination typologies.

Another important aim of the invention is to provide a control system for a magnetic resonance scanner allowing a simple management by the staff responsible for the system (sequence developers and medical staff) and simple management of data acquisition, therefore making the machine control and management as much as possible transparent to the user.

The technical task mentioned and the aims specified are achieved by a control system for magnetic resonance scanners as claimed in the appended Claim 1. Preferred embodiments are highlighted in the sub-claims.

The features and advantages of the invention are hereinafter clarified by the detailed description of a preferred embodiment of the invention, with reference to the accompanying drawings, in which:

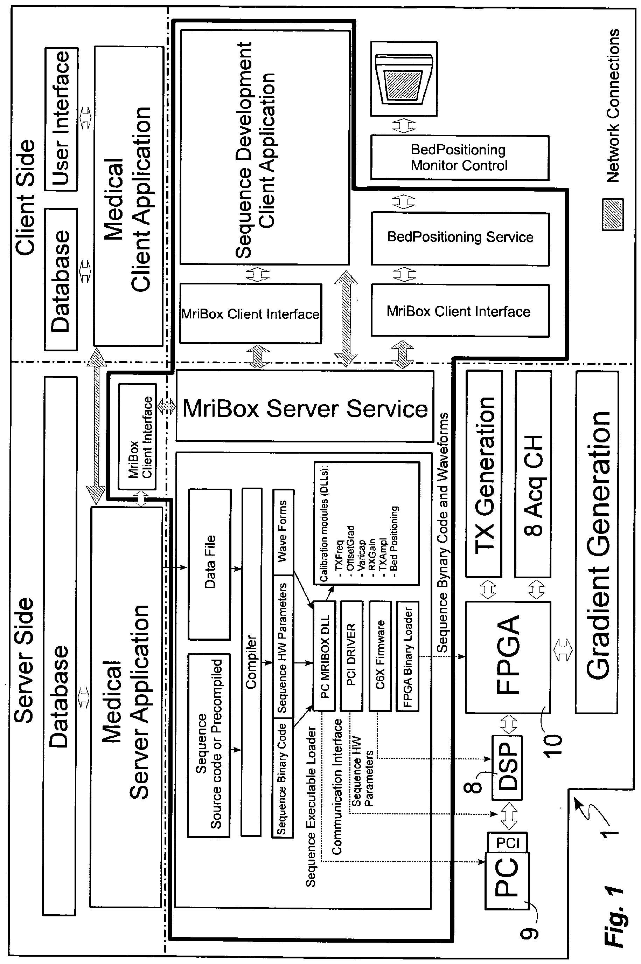

**Fig. 1** shows a first operation diagram of the control system according to the invention;
**Fig. 2** diagrammatically shows the scanner operated by the control system in accordance with the invention;
**Fig. 3** is an operation diagram of part of the control system according to the invention;
**Fig. 4** is a further operation diagram of part of the control system according to the invention.

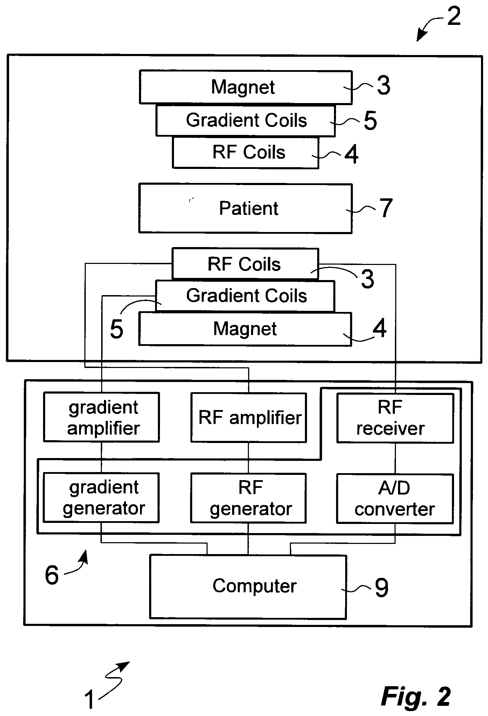

With reference to the mentioned figures, the control system for a magnetic resonance scanner according to the invention is generally identified with reference numeral **1.** It is adapted to regulate a scanner **2** suitable to produce magnetic resonance images or tomographies, or merely MRI images. Scanner 2, diagrammatically shown in Fig. 2, is mainly formed with elements producing static magnetic fields or magnetic fields varying in time and space, fundamentally consisting of a main magnet **3,** the function of which is to create a static and uniform high-intensity magnetic field, radiofrequency or RF coils **4,** generating a Larmor-frequency rotating magnetic field, gradient coils **5** generating magnetic fields linearly varying in space, that are indispensable to image generation. They are placed in the vicinity of a bed **7** for a patient. Scanner 2 further comprises a spectrometer **6.** Said spectrometer attends to management of scanner 2 enabling the necessary signals for control of the magnetic field to be generated, and in particular it generates and controls RF (radiofrequency) pulses and gradients and allows full control of same as regards their features.

Said signals generated by spectrometer 6 are amplified by apparatuses external to the spectrometer and sent to the gradient coils 5, RF coils 4 and magnet 3.

Therefore system 1 carries out management of the spectrometer 6 and acquisition and saving of data relating to the RF signal received from the coils. Further provided is a series of automatic calibrations allowing quick setting of scanner 2 and system 1, depending on the features of magnet 3 and of the patient under examination.

The course of the events that are necessary to achieve operation of system 1, and in particular control of spectrometer 6 regulating the magnetic field, is referred to as sequence and consists of a series of API (Application Programming Interface)- function calls describing the type of examination or test that the machine will have to execute.

The sequences determine particular examinations that can be very different from each other, the complexity of which will also vary to a great extent.

They can be implemented by the responsible staff, typically physicists, that starting from theoretical bases, implement the examinations or sequences that the scanner 1 will have to execute, therefore establishing which signals are to be generated as well as the acquisition of the received data that will then bring, following mathematical processing operations, to the MRI image. The medical staff will then be able to make a selection among the different examinations previously programmed, through a suitable graphic management interface.

The spectrometer is in particular controlled by an electronic processor including mutually interacting hardware and software devices.

In particular, the software consists of many modules connected to each other and mutually interacting, which all together allow use of spectrometer 6.

Each module implements specific functions linked to the underlying hardware to a different extent.

The implemented modules respectively are (in an increasing order the layers that are gradually farther from the hardware): DSP Firmware (C/C++), Windows driver in Kernel Mode, Windows Driver Foundation (KMDF) (C), main DLL of control system (C/C++), automatic calibration DDL of the system (6 modules) (C/C++), sequence structure (API) and compiler (C/C++), main Windows service for system control and management through network (MRIBox Server Service (C/C++), module of TCP client connection (MRIBox Client Interface) (.NET 2.0 C#), Windows service for bed positioning (Bed Positioning Service) (.NET 2.0 C#), client application for development and MRI sequence debug (.NET 2.0 C#).

All subsequently described software modules contribute to correct operation of scanner 2. The whole software can be defined as a software layer interfacing with the hardware of scanner 2 and with two management interfaces. One of them comprises an image reconstruction portion, a database and a graphic management interface of the machine for the medical staff responsible for use of the machine itself. The other, dedicated to the development of examinations (or sequences) is conceived for meeting the requirements of the MRI developer.

In greater detail, the DSP (Digital Signal Processor) firmware **8** is the first level of implemented software which is the closest to the hardware.

The DSP firmware 8 is interfaced with a PC **9** or computer or the like and in particular with a PCI controller.

The DSP firmware 8 has an interrupt management of the vectorial type (and therefore a completely deterministic management) and allows latency problems typical of an operating system to be avoided, by directly managing the critical events from a time point of view and offering the possibility of relaxing the maximum latency values on the PC 9 side.

On one side it interfaces with a FPGA **10** (field-programmable gate array) controlling the spectrometer 8 and on the other side directly with the PC 9. The FPGA 10 is the device on which physically the sequences are executed. In the firmware of the FPGA 10, in fact, a microcontroller dedicated to sequence execution is implemented and the type of code to be loaded in the microcontroller is a binary code consisting of macroinstructions that directly control the hardware operations. The algorithms that can be implemented have a strictly sequential character with the execution times fixed by setting delay parameters, which delays is specified as a parameter in each individual macroinstruction. There is the only possibility of implementing loops also nested with a programmable number of iterations. However status registers or indexes of the implemented loops are not available. The sequence loaded in the FPGA 10 is in the form of a binary code. This is carried out with rigid execution times that are defined by merely scanning the sequence of events imposed by the sequence operator with predetermined times.

A compiled sequence not only consists of this binary code but also of a series of other additional elements that are of help thereto. For instance, frequency-jump tables for multislice sequences, tables for the gradient rotation matrix, the waveforms of the gradients and the RF pulse, acquisition parameter setup and still others.

Among the main tasks of the DSP firmware 8 there is the initialisation of the whole system 1 at the start and, at the moment of loading the sequence, the task of carrying out the setup of the whole hardware configuring it for execution of the loaded examination.

Once the sequence has been loaded, when it is being run following a command by the user in the graphic interface, the FPGA 10 begins generating the commands controlling both generation of the signals and acquisition of the feedback interrupts to the DSP firmware 8.

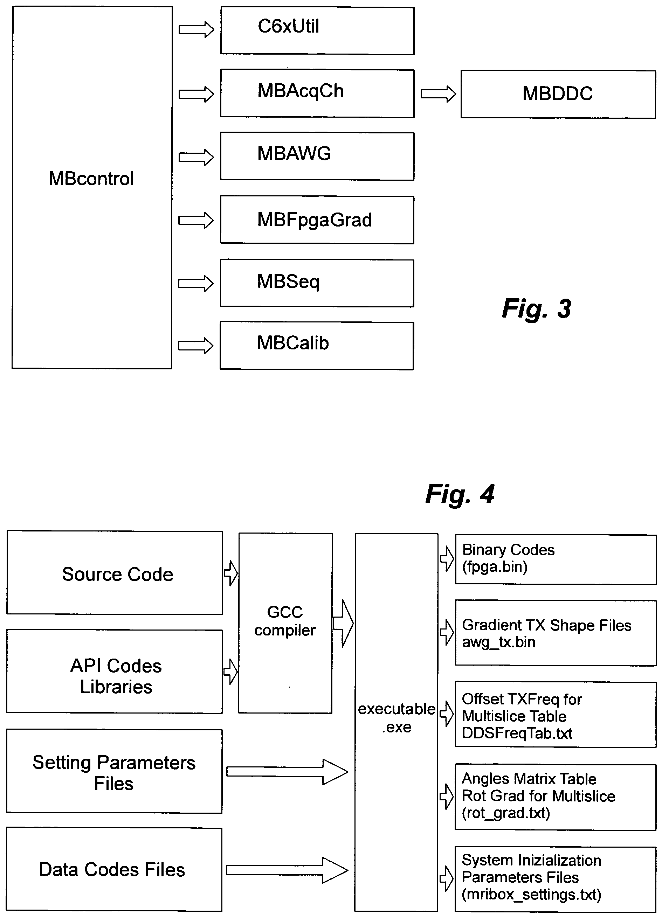

Generation of these feedback interrupts is automatically inserted during the compiling step (i.e. conversion from the programming APIs of the sequence to the binary code, Fig. 4) within the sequence itself (therefore they are programmed by the same sequence operator even if not explicitly).

In addition, the DSP firmware 8 controls updating of the transmission frequency values and the angles of the gradient rotation matrix in case of multislice sequences. In fact, in this case a suitable sequential instruction causes the DSP firmware 8 (through generation of interrupts and messages), during execution of the sequence itself, to read the new values of transmission frequency and rotation matrix from the specific tables (generated by the compiler) and then directly setting them in the sequence memory in the operands of the frequency-change binary instruction, in case of the transmission sequence, or in the suitable registers for the gradient rotation matrix.

Also implemented in the DSP firmware 8 are the drivers of lower level for control of the machine hardware.

All the parameters of the system are packaged in a data structure initialised by the PC 9 either during the sequence loading step or alternatively through specific configuration messages.

Also implemented in the DSP firmware 8 are control functions of the hardware that can be recalled from the outside of the DSP firmware 8 and therefore the PC 9 through a PCI bus, with the aim of a sequence debug, offering the user the possibility of setting and imposing values to system parameters in real time in the development interface or, through APIs, within automatic calibration modules, providing the software tool for closing the control loop of the implemented calibration algorithm.

Some of these parameters are configured by merely writing a value in the appropriate programming register, others such as the transmission frequency are set at runtime by dynamically modifying the operand of the frequency-change instruction in the binary code of the sequence loaded in the microcontroller implemented in the FPGA 10.

In addition, since within the controller implemented in the FPGA 10 it is not possible to have indexes of the loops relating to the iteration number, this is counted for each loop by the DSP that in this manner has the sequence execution state at its disposal so that the indexes required for management of data transfer and other functions are available.

The DSP firmware 8 also carries out management of the acquisition. The acquired data coming out of a receiver 10 comprising a Digital Down Converter, divided for each channel in the form of real and imaginary part are stored in a temporary buffer. They are data in a complex form as they come from a quadrature step demodulation done within the Digital Down Converters of the reception channels.

The DSP firmware 8 then controls the acquisition channels by loading in the appropriate devices the gain parameters being received, voltage on the varicaps for tuning of the reception coils, sampling frequency coming out of each Digital Down Converter, loading of the digital filter coefficients of the Digital Down Converters and all that which is further necessary for acquisition. These data are then transferred to a circular buffer implemented in the DSP and then sent to the PC 9 through a DMA PCI Bus Mastering transfer.

Before describing operation of the circular buffer in detail it is necessary to briefly explain some features of the data acquisition inside an MRI sequence. Depending on the setup of the sequence set from the user interface, the number of samples of each acquisition, or encoding, can vary in a range of 0 to 4096.

In addition, the acquisitions in any sequence that can be implemented are typically inserted in a nesting within various loops or iterations. These iterations, up to five, are referred to, according to the nesting order (from outside to inside) as: 3d-codings, codings, excitations, slices, echoes.

Also the maximum number of each of these iterations is a fundamental parameter of any sequence exactly as the number of samples. All these maximum values as well as the number of samples are, as a rule, constant during execution of the sequence.

These loops are part of the general structure of a sequence and allow an acquisition to be identified through the indexes of the iterations in which it has been done, being at all events linked to the time execution of the sequence itself. These indexes are implemented in the DSP firmware 8 and are updated through sending of interrupts and specific messages by the FPGA during execution of any sequence.

In implementing the circular buffer in the software of the DSP, four ranges can be distinguished in the variation range of the number of samples. The ranges are: 0 to 256, 257 to 512, 513 to 1024, 1025 to 2048, 2048 to 4096.

Then five values of maximum size of an element to be transferred have been considered, i.e. 256, 512, 1024, 2048 and 4096. Therefore if a sequence in the setup has a number of samples lower than or equal to these five thresholds, from the point of view of data transfer the maximum amounts of elements within this range will be in any case transferred. In the control DLL then the real number of transferred samples will be taken into account.

To each of these data blocks of a fixed size a header is added that describes the coordinates thereof within the sequence, i.e. the status of the various indexes at the moment the acquisition has been made plus the acquisition channel and the number of samples. These numbers uniquely identify the acquisition fixing the time instant at which it has been made through the values of the loop indexes.

These data blocks made up of a header and the acquired samples are stored in the circular buffer in the DSP memory. The sizes of this circular buffer are varying and determined by the size of the blocks to be transferred into the previously described five bands. This buffer in fact is allocated considering the base element to be transferred depending on the number of acquired samples. Indexing of the buffer takes place therefore at block level and not at byte level where the block size is varying depending on the amount of acquired data as previously described.

The circular buffer thus instanced (dynamically on loading of each sequence) is managed through two pointers (or indexes), a reading one and a writing one. Increase and decrease of these indexes is done through the interrupt and acquisition message by the FPGA 10 (and therefore the sequence) for the writing pointer and through data sending on the PCI bus for the reading pointer.

Sending of data on the PCI bus is fully managed by the DSP firmware 9 in the software of which also the PCI communication protocol is implemented with management of the Transactions, Transfers and management of possible retransmissions in case of errors. The policy of sending data is not immediate. The amount of data blocks sent on the PCI bus at each DMA transfer can be defined as a parameter of the sequence. In this manner it is possible during the sequence debug, to transfer the amount of data necessary to have the possibility, by the graphic tool of sequence development, of graphically navigating in real time within the acquired data (in blocks of a defined sequential size). For instance, if a sequence with the following features is considered: 3d-codings = 1; codings = 256; excitations = 1; slices = 24; echoes = 1; number of channels being received = 2, deciding on transfer of 48 DMA event blocks to the PCI bus, all the 24 slices of the two channels will be at the disposal of the graphic client for being displayed and analysed in real time by navigating through the acquired data with the suitable tools. In this manner it will be possible for the graphic development tools to take advantage of a sort of trigger synchronised with the acquisition parametrizable by sequence.

In addition, this expedient allows implementation of very fast sequences, in the order of milliseconds, i.e. with times that could be hardly managed directly by a PC due to the latencies of the non-strictly real-time operating system. Since more than 1000 data blocks can be acquired in the DSP memory (up to 256 samples) it is possible to implement sequences with very narrow times, managing the events with the vectorial DSP interrupts and therefore in a deterministic manner, accumulating the data in the circular buffer for then transferring them, even at the end of the sequence, into the host PC memory. An example of use is the calibration sequence of the bed positioning in real time where an MRI image (made up of 128 acquisitions and 128 samples) is acquired in times on the whole much smaller than one second.

Another advantage resides in the calibration modules where in addition to a specific sequence generating the data to be processed there is an algorithmic part at the inside of each specific calibration DDL processing the acquired data and closing the control loop acting on the physical size to be calibrated. By suitably managing the number of blocks to be transferred it is possible to synchronise starting of the algorithmic processing with the event of acquisition of the data blocks, i.e. enabling the processing to be carried out in real time during execution of the calibration sequence itself.

The amount of the blocks to be transferred, defined in the code of each sequence, can be fixed by setting it with a predetermined value before compilation of the sequence or it can be parametrized at will by automatically calculating the value thereof through use of parametrized formulas through the sequence parameters.

System 1 further comprises a KMDF Windows driver managing data transfer on the PC 9 side and message exchange for which however it is actually transparent. Arriving at the Windows driver are the interrupts and messages generated by the DSP during the PCI Bus Mastering transactions and during the normal message exchange. Allocated in the Windows driver in the physical addressing space of the PC memory 9 are two exchange Common Buffers between user and kernel mode the base pointers of which (physical-address pointers in kernel mode) are passed to the DSP firmware 8 to supply it with the physical base addresses of the buffers on which data are to be transferred. Management of these buffers takes place in ping-pong, i.e. the two buffers are exchanged at each data transfer. When one of the two buffers is full, its corresponding base address mapped in the virtual addressing space of the process of the control DLL in user mode is passed through an event generated by the driver itself to the control DLL that will carry out management of the acquired data. The pointer to the physical address of the other buffer (the empty one) will be simultaneously sent to the DSP for subsequent transfer. In addition to the pointers to the two buffers in the virtual addressing space, the pointer to a memory area used as a signalling flag is passed to the control DLL in user mode for letting the control DLL know in user mode which of the two buffers is active at a predetermined instant.

In addition to the above, the driver supplies the control DLL in user mode with the elementary reading writing memory functions and generation of interrupts to the DSP firmware 8 that are necessary for system operation as well as management of bi-directional message exchange between control DLL and DSP firmware 8. The control DLL, diagrammatically shown in Fig. 3, is a sort of high-level driver enabling the underlying hardware to be abstracted and allows interfacing with the system through a defined API Set and an assembly of configuration files. Together with the compiler (from which it receives the binary code and the operation parameters generated by compilation of a sequence), it actually constitutes the separation line between the closest part to the hardware and the control and interfacing part of the system. On one side it interfaces with the driver and the DSP and on the other side with the control service. It further interfaces, as said, with the sequence compiler since the compiled sequences are loaded in the spectrometer hardware from this DLL, to be then executed. The whole passes through this DLL module, from the system start to the download of the DSP firmware (carried out by the PC during configuration), to sequence loading, up to management of the system parameters even during the sequence runtime.

Implemented in this module are the necessary data structures, the parsers for reading the files of the compiled sequence, the control APIs of the system parameters as well as additional functions of tests and debugs.

All the configuration parameters of the eight reception channels are packaged into two specific classes MBDDC and the daughter class MBAcqCh, as diagrammatically shown in Fig. 3. Through eight instances of the last-mentioned class (one by each reception channel), full control of the data acquisition system is obtained. The aim of this infrastructure is management of the whole acquisition from the analog part to the demodulation, filtering and undersampling part implemented by the eight DDCs (Digital Down Converters) placed immediately downstream of the samplers.

The DSP control part is implemented in the appropriate class C6xUtils managing the DSP from the firmware loading to exchange of system configuration messages.

The loading control and sequence execution is managed by class MBSeq which also has the function of sending the DSP the information concerning the stored offsets of some types of macroinstructions of the binary code of the compiled sequence (generated during compilation), for instance to enable management of the frequency jump for the multislice sequences by directly modifying the operand of the macroinstruction in the binary code.

Loading and initialisation of the RF pulse and gradient generator is managed by class MBAWG that sends the DSP and related HW the parameters and waveforms generated by the compiler. The waveforms will be then used for generating the gradient and RF signals depending on the commands present in the binary file of the loaded sequence.

Control of the hardware of gradient post-processing with preemphasis filters for compensation of the Eddy Currents is implemented in module MBFpgaGrad. Also implemented herein are the gain management APIs, DC offset and gradient delay.

In addition to the previously listed classes, the abstract class MBCalib constitutes the interface to the different DLLs containing the calibration classes.

All these classes interact with class MBControl supplying the control APIs that enable interfacing with the Main Server Service.

This module further carries out management and saving of the acquired data. Data are stored in a multidimensional buffer dynamically allocated depending on the specific features of the sequence to be executed (number of active channels, 3d-codings, codings, excitations, slices, echoes).

At the end of the sequence (and consequently of the acquisition) the stored data will be saved on a file in a format compatible with the image reconstruction algorithms.

The data transfer event within the control DLL is generated by the driver each time a buffer is available.

Following generation of the event, within a data receiving thread, it is determined to which block size the transferred data (256, 512, 1024, 2048, 4096) refer. When the type of data block has been established, it is determined which buffer (ping or pong) is active, following verification of the specific flag made available by the driver. This step terminated, the software goes on copying the acquired data inside a multidimensional buffer containing all acquisitions relating to execution of a sequence. The data are disposed in the buffer depending on the indexes contained in the header (initialised by the DSP during execution of the sequence) of each transferred block.

Should the calibration modality be activated, the received data will be made available within the calibration module instanced through the appropriate API. In addition the specific callbacks will be called for synchronisation of the execution of the calibration algorithm instanced with the acquisition events generated by the specific calibration sequence.

System 1 further comprises the automatic calibration DLL modules (Calibration Modules). They provide the possibility of calibrating the main operation parameters. In particular six calibration modules are present that regulate: calibration of transmission Frequency (CalibTXFreq.dll), calibration of gradient Offsets (CalibOffsetGrad.dll), calibration of pulse Amplitude 90 (CalibTXAmpl.dll), Varicap calibration of reception coils (8 channels) (CalibVaricap.dll), calibration of reception Gain (8 channels) (CalibRXGain.dll), calibration of bed positioning (CalibBedPositioning.dll).

Each calibration consists of a dedicated sequence (referred to as calibration sequence) generating signals and managing data acquisition, and of an algorithmic module (a DLL) processing the acquired data and, if necessary, closing the control loop by varying the parameter under calibration depending on the processing results.

Start of a calibration, carried out through the graphic interface, puts the system in a specific operation mode because the simple storage of the acquired data and consequent saving of same on file will be no longer carried out, but an iteration will be started enabling development or treatment of the necessary calibration operations. The result of each calibration will be saved on a specific calibration file that will be then used by the compiler for all subsequent sequences compiled, so that all system parameters will be updated. Execution of a calibration comprises management of subsequent steps involving compilation, loading and execution of the calibration sequence. This series of operations is managed in the Client DLL module through specific threads that by managing the events generated by the system allow execution of the calibrations to become automated.

The six calibration modules are daughter classes of the MBCalib abstract class (containing virtual functions as well) implemented in the control DLL module. Each calibration class inherits the callbacks relating to the sequence events (start, stop and acquisition) from the mother class and in addition inherits a large set of control APIs for management of the system.

In greater detail, the general infrastructure of a calibration module consists of five methods: two for constructor and destroyer, and three others for callbacks of the execution events. Through the three callbacks it will be possible to have the feedback of the main events relating to execution of the calibration sequence.

In addition to these callbacks there are three different sets of APIs. The first one is dedicated to control of the machine hardware (in case of calibration of the varicaps, for instance control of the outgoing voltage), for a total amount of 36 APIs. The second is dedicated to control of the sequence execution (for instance, offset management of sequential macroinstructions, etc.), for a total amount of 20 APIs. The third is dedicated to additional operations relating to calibrations (for instance, reading and saving of parameters on files, access to acquired data, etc.) for a total amount of 76 APIs.

These APIs are made available within the calibration classes through three pointers to tables (one for each set of APIs) with member functions.

This API set allows overall management of the system inside each calibration module therefore making each module very flexible as regards the operations that can be implemented and managed.

The algorithmic module of each calibration sequence must therefore be implemented inside a DLL which must contain a daughter class of the abstract class MBCalib from which it will inherit all the callback and API set made available by the system for implementation of the calibration algorithms. Among the APIs made available there is a set of functions for management of reading and writing of the calibration values on the saving file and the setup values of the algorithms on the general calibration configuration file. These files are based on use of some parsers that during the calibration initialisation step access the configuration files and initialise some system structures making available the preceding or initial calibration values, through APIs. When calibration has been completed, other APIs enable saving of the new values found that will be then used by the compiler upon compiling a new sequence.

There is also a function that during development of a calibration algorithm offers the possibility of sending data and number strings directly to the control service and the development client for being displayed in an appropriate log window with debug purposes.

Calibration of the varicaps (together with calibration of the gradient offset) has the following limitation: due to the present hardware implementation of the system, access to the register managing the varicap voltage (and the value of the gradient offset) is not possible during execution of the sequence.

This involves the necessity to carry out individual iterations of the calibration sequence and a need for management of the sequence restarting from the inside of the algorithmic calibration module between an iteration and the following one. In detail, the event triggering data processing is no longer the acquisition but the end of the sequence running.

Due to this, the algorithm, following the decision of going on processing, must be able to inform the system that it will have to restart the sequence.

As regards calibration of the varicaps and the gain being received, there are further features to be taken into account. Calibration must be carried out on several channels in a simultaneous manner. Channel selection takes place from sequence (or data file) through activation or deactivation of an acquisition channel.

The algorithm must therefore have the context allocated separately for each instance (by context it is intended all variables determining the status thereof between an iteration and the following one).

Each context must be dynamically allocated (malloc) in the constructor or more simply put in the .h file of the calibration class as a member. This data structure is implemented in the form of array for facilitating access thereof through indexing with the channel number. In this way, at the calibration start, at the constructor call, all contexts will be automatically instanced. Each instance will be then assigned to an active channel and will pick up its data from the buffer with the correct channel offset.

Data reading of the preceding calibrations and saving of the new calibration values on file must be managed through the appropriate APIs enabling access to the indexed calibration file, this one too with the channel number.

Since it is not possible to impose the same iteration number of the algorithm to all instances of the active channels because each of them has an evolution of its own that is independent of the others, use of a flag array is necessary for managing the exit condition of the search algorithm for each instance. Each instance must lift a flag on this array at the end of the operations of that instance itself and the overall exit condition will be a logic AND of the flags of all active channels. It is further provided a timeout defined with a maximum number of iterations through setting of a maximum number of sequential iterations (calibration of the gain being received) or through a configuration parameter of the algorithmic calibration module (varicap calibration).

By this infrastructure it is possible to manage the calibrations both in a simultaneous manner (several channels calibrated in a single execution or running) and in a separate manner for each channel. In fact, to the higher level (i.e. the graphic software management) it will be possible to call the calibration several times on a single active channel in turn so as to make the calibration procedure of the varicaps serial and independent. However for this option an execution time that is more than eight times longer (taking into account the eight channels) is required, due to clear implementation reasons. The calibration assembly controlled by sequence: Calibration 90 (RF Pulse), Calibration of the transmission Frequency and Calibration of the Gain being received has a much more linear operation that than with the sequence restart (gradient offset and varicap). In this calibration assembly in fact, the sequence fully manages the algorithm iterations. The maximum number of iterations is established in the data file by the maximum number of codings that the calibration sequence will have to execute. Data are made available inside the calibration module through appropriate methods.

Calibration can also terminate in advance if the algorithm has reached the value to which it must converge before the maximum iteration number. For frequency and RF pulse calibration, further appropriate APIs are present that provide the macroinstruction offset in the binary file of the sequence to be modified with change of the operand of the macroinstruction containing the transmission frequency or pulse amplitude. These parameters would be otherwise inaccessible with the sequence being executed.

Calibration of the bed positioning is also controlled by an appropriate DLL module. Unlike the preceding ones, it has no feedback loop. It merely acquires data generated by a very fast acquisition sequence capable of generating an image (128 codings x 128 samples) in periods of time lower than one second. It has a direct access to the acquired data buffer. At each acquired image the raw data are coupled in a temporary buffer, a bidimensional FFT thereof is made and the values thus calculated are normalized to 1. At this point the thus processed image is sent through the control Server Service to all the network clients at that moment connected through the TCP socket. In addition to this, through the bed positioning service interfacing with the Client DLL, it is possible to connect the system with an LCD remote monitor with a touch screen positioned close to the magnet.

This monitor enables the medical staff to have a feedback during positioning of the patient thus facilitating and shortening the preparation operations for the examination. Through the monitor it is not only possible to display the image, but also to operate two start stop sequence commands to manage execution of the film and select the values of the gradient rotation matrix between the coronal, axial and sagittal one. All control messages are managed by the Client DLL.

The compiler, diagrammatically shown in Fig. 4, is the other module that, together with the control DLL enables the underlying hardware to be abstracted making the functions thereof manageable. Programming of a sequence at the present state is done by implementing a C code using a specific library of APIs having the function of building (after the compilation step of the C source code and execution of the generated executable) the binary file of the sequence. Defined as the sequence compiler is actually a compiler without the lexical analysis part, i.e. the parser (the GNU Bison, for instance). The sequence is directly written in C and is inputted to the GCC compiler together with the related Data File and the calibration file. This has been done for having a greater flexibility of the system also for future developments relating to a possible implementation of the sequence by graphic tools to be made by integrating them in the sequence development environment. From the sequence source code and the development API libraries the compiler generates an executable. This executable in turn generates the binary file of the sequence to load in the microcontroller implemented in the FPGA completed with all data files and supporting parameters.

The sequence development APIs are such structured as to enable implementation of the sequence using the features made available by the underlying hardware.

These APIs allow all the system functionalities to be controlled. Each API corresponds to a specific macroinstruction completed with its configuration parameters. During execution of the executable generated by the compiler, each API writes, in the sequentially defined order, the corresponding macroinstruction in a file in the binary format (fpga.bin). The macroinstructions following one another in the sequence source code determine generation of the binary file to be executed in the microcontroller implemented in the FPGA 10.

The last parameter of each instruction is a delay value for the following instruction. The sequence execution is absolutely deterministic and linked to the delays defined during the implementation step.

The compiler also takes into account the macro index so as to set suitable variables that, passed to the control DLL and by turns to appropriate DSP tables, will enable management of the transmission frequency jumps for multislice sequences, as well as execution of the commands necessary to the algorithms for calibrating frequency and amplitude of the transmission pulse.

In addition to the above, the compiler carries out parametrization of the sequence and generation of the gradient waveforms. In fact, a sequence is made up of three distinct parts.

A first part consists of the parametrization part with the sequence control formulas: this part inputs the data file enabling, through calculations studied at the sequence planning moment, parametrization of parameters such as, for example, number of slices, slice thickness, distance between a slice and the following one, maximum number of iterations (3d-codings, codings, excitations, slices, echoes), number of samples to be acquired, etc.

A second part is the true sequence containing the hardware events to be generated taking into account the parameters (among which the delays) calculated in the preceding block.

A third part consists of the gradient and TX generation part which carries out generation of the waveforms relating to the three gradients and the transmission pulse. The amplitudes of these gradients will be linked to the calculations made in the parametrization part while the signal duration (signals generated with a constant sample number) will be adapted by the hardware to the duration of the pulse established by the sequential commands.

Also present is a main server for control and management of system 1 through the network.

It supplies system 1 with the network interface enabling remote control thereof and interfacing with the various, medical and additional, development applications.

The communication is based on two sockets: a TCP and a UDP socket. The first is dedicated to the machine control for use not during development of the sequences and can be managed on the client side through the TCP Client connection module. The second is dedicated to the additional functionalities for development and allows through the Client application for development and the MRI sequence debug (in combination with the TCP connection), a series of tools to be made available for analysis in real time of the acquired data and management of the system parameters also in real time during execution of the sequence.

Through the TCP socket different functionalities are present: management of the sequence compilation (with possibility of having the compilation log); management of the calibration execution (compilation, loading and execution); sequence loading; sequence execution and stop; additional control functions; general system debug and log functions; management of system events.

The TCP Socket supports multiple and concurrent connections of the Client module enabling simultaneous use of the MRI sequence development Client, the medical interface application and the Client for patient positioning.

The UDP Socket allows use of the system only by the MRI sequence development Client being dedicated to sequence development and debug.

Among the additional functionalities of the UDP Socket there is the control in real time of the following parameters: gain of the reception channels, voltage value for varicap of reception coil tuning (8 channels); values of frequency offset in the frequency jump table for multislice; values of gradient rotation matrix; digital gain on reception; gradient offset values; and even multiple sending of slices and echoes (up to five simultaneously) coming from the acquired data with the possibility of selecting sending of the acquisitions of interest through setting (still through the socket) of control indexes connected to the sequence features. Control in real time of these parameters takes place during execution of the sequence and during display of the acquired data so as to have an immediate feedback by the system.

The transferred data will be then displayed on the sequence development Client interface. Note that the transferred data in this manner are not directly connected to the acquisition thread of the control DLL. In fact, to make the two activities completely independent, a second thread parallel to the acquisition thread but with a normal priority verifies the data arrival in the accumulation buffer and, when they are completely available, allows transfer of the selected acquisitions.

This is done in such a manner as to fully release the time-critical acquisition part from the part concerning data verification and sending to the development client that, being an additional function, does not have the execution constraints of the acquisition. These expedients and this multithread implementation are made necessary for combining, on the one hand, the system reliability during acquisition (constraints of acquisition times) and, on the other hand, the possibility of having an assembly of data displaying tools in real time for analysis of the sequence being executed that is reactive to the user's settings.

The TCP Client connection module is the client DLL module enabling remote control through the network connection. Through this module associated with some network-shared folders on the Host PC (containing the sequences, parameter files and other configuration files) the remote control of the system is possible in all the functionalities thereof. The module also supplies the paths of the different files and folders necessary for the system management.

This software module after a first connection step enables the following functionalities to be managed: management of the sequence compilation (with the possibility of having the compilation log); management of calibration execution (compilation, loading and execution); sequence loading; sequence execution and stop; additional control functions; general system debug and log functions; management of the system events.

This client module can be connected to the server both locally on the host PC and remotely. It represents the main interfacing module to the whole system and has been planned in such a manner as to make the multiple connection with the system control Server Service possible. It is used both in the sequence development client and in the physician interface, as well as in additional functions such as the remote monitor for patient positioning. Through use of delegates it further allows feedback from the system to be obtained, so that the events generated in the normal use of the machine can be managed.

In greater detail this assembly contains a TCP Client and an assembly of other elements allowing the system management. The assembly is made up of an assembly of methods and delegates.

Through these methods it is possible to control the whole system first opening the TCP socket (specifying IP address and port of the remote Server machine) and subsequently managing all the functionalities necessary for the machine operation. The DLL further supplies the paths too and the names of all the configuration files for facilitating identification of same during the data and parameter reading writing steps. The .NET delegates provide the means for management of the events coming from the remote system.

The development Client application is the interface for the sequence developer. It consists of a .NET windows application implemented in C# and packages and makes it possible to manage the whole software architecture and the related previously described hardware. It is planned for being a complete development environment for MRI sequences and the type of infrastructure is Multiple Document Interface with the classic basic tools for editing text files. Operation of the interface is related with the communication with the main control Server Service to which it is connected through two sockets, one of which is a TCP socket for the operations for ordinary management of the system (through the TCP Client connection Module) and the other is a UDP socket managing the advanced management operations of the system and sequence debug operations. Through this interface after start of the network connection with the server, it is possible to open a sequence (exactly as if it were a software development project), open the files relating to the project that are present in the tree to the left of the main window and possibly modify them, select a data file with the sequence parameters and subsequently compile, load and put the sequence to execution.

On starting the sequence execution automatically after transfer of the first coding block, the server for each activated channel will send part of the acquired data for real time display.

The user, depending on the sequence features, can navigate through the acquired data selecting, by suitable controls, the slice, first echo and total number of echoes (simultaneously maximum 5 for slice and for channel) to be displayed in real time during acquisition.

These parameters will be sent to the server that at the next data sending (the following block) will provide for sending of the requested data. The activated windows will be connected to the sequentially activated channels, one for each channel. Display of the real and imaginary part and of the acquired data module is available for each channel.

In addition the user, through the appropriate interface, will manage change in real time of some system parameters being able to change system parameters even during running of the sequence or, if the user prefers it, by test when the sequence is not running.

When acquisition has been terminated, once the data have been saved on file, it is possible to open the acquired-data file (when the sequence is not running) through a specific tool included in the graphic development environment and navigate through all the acquisitions with the possibility of carrying out measuring on the acquired data and save them in text format. It is further possible to reconstruct an image starting from the acquisition coordinates thereof (channel, 3d-codings, excitation, slice, echo), display one or more images with functionalities such as zoom, value measuring and management of the image level (clipping of the higher levels and raising of the underlying background noise).

This tool has proved to be a very useful one because it allows an accurate measuring to be done on the acquired data and further enables a first reconstruction (based on a FFT 2D) to be done that allows a feedback on the acquired image to be immediately available.

As said, put beside each sequence there is at least one data file parametrizing operation and features thereof. The parameters of the data file can be displayed and modified through a graphic interface in a specific graphic tool. In addition to the above, the interface allows management of the system calibration parameters. Once a sequence has been opened and the data file selected, in fact, it is possible to start the individual automatic-calibration algorithms. Execution of a calibration comprises compilation, sequence loading and running, all that managed automatically by the system and linked to the only pressure of an appropriate pushbutton on the interface. The calibrated data will be saved in a specific calibration file that will be used by the compiler for all following sequences. In addition the values present in the calibration file can be displayed and modified within the same graphic interface.

A particular speech is to be made for calibration of the bed positioning. This calibration, once triggered by the pressure of the appropriate control pushbutton and after the compilation, loading and execution step, starts a series of repeated image acquisitions. Reconstruction of each image (done in real time within the algorithmic calibration module) is received by the Client application (through the specific event of the TCP Client DLL) and displayed in a graphic window. Also present in the same window are the film start and stop pushbuttons managing execution of the sequence.

In addition, from this interface it is possible to control selection of the gradient rotation matrix to be used (coronal, axial, sagittal one). The same functionalities and control are simultaneously available in all clients connected at that moment, obviously inclusive of the remote LCD module positioned in the vicinity of the magnet that will enable the medical staff to position the patient in the easiest manner.

Management of the remote LCD module relies on the bed positioning service supplying the MRI image displaying function in real time, being interfaced on one side with the TCP client module and on the other side with the monitor management module. This service is automatically connected, on starting of the system, to the main control server service, and keeps waiting until starting of the bed positioning calibration.

In addition, it offers the functionality of film start stop and selection of the image to be acquired through change of the gradient rotation matrix (coronal, axial, sagittal one).

Therefore the described graphic client application not only manages the basic tools for sequence programming, but also manages a series of tools addressed to the sequence developer (or sequence operator).

The described software mechanisms, in data transferring from DSP to the control DLL and in the system control methods, allow a better management and display of the acquired data to be obtained in real time, enabling the sequence developer to carry out a simplified analysis, depending on the sequence type to be developed.

## Claims

1. A control system for a magnetic resonance scanner suitable to manage the production of magnetic fields by said scanner and the acquisition and saving of data relative to the signals received by said scanner, comprising an electronic processor including a software suitable to control said scanner and comprising a graphic development environment suitable to provide basic tools for the choice of examinations and the acquisition of images, and **characterised by** comprising a DSP firmware (8) controlling updating of the transmission frequency values and the angles of the gradient rotation matrix in case of multislice sequences, said DSP firmware (8) being adapted so that a suitable sequential instruction causes said DSP firmware (8), through generation of interrupts and messages, during execution of the sequence itself, to read the new values of transmission frequency and rotation matrix from the specific tables generated by the compiler, and then directly set them in the sequence memory in the operands of the frequency-change binary instruction for the case of the transmission sequence, or in the suitable registers for the gradient rotation matrix.

2. The control system according to claim 1, wherein said graphic environment comprises a series of tools suitable to allow programming of said examinations.

3. The control system according to one or more of the preceding claims, provided with Client/Server architecture.

## Patentansprüche

1. Steuerungssystem für einen Magnetresonanzscanner, das geeignet ist, die Erzeugung von Magnetfeldern durch den genannten Scanner und das Erfassen und Speichern der von dem genannten Scanner empfangenen Daten in Bezug auf Signale zu verwalten, umfassend einen elektronischen Rechner einschließlich einer Software, die geeignet ist, den genannten Scanner zu steuern, und umfassend eine grafische Entwicklungsumgebung, die geeignet ist, grundlegende Instrumente für die Auswahl von Untersuchungen und die Bilderfassung zur Verfügung zu stellen, und **dadurch gekennzeichnet**, eine Firmware DSP (8) zu umfassen, die die Aktualisierung der Werte der Übertragungsfrequenz und der Winkel der Rotationsmatrix der Gradienten im Fall von Mehrschichtsequenzen steuert, wobei die genannte Firmware DSP (8) so ausgelegt ist, dass eine geeignete Anweisungssequenz dazu führt, dass die genannte Firmware DSP (8) durch Generierung von Interrupts und Meldungen während der Ausführung der Sequenz die neuen Werte der Übertragungsfrequenz und der Rotationsmatrix aus den vom Kompilierer generierten spezifischen Tabellen abliest und diese dann direkt im Sequenzspeicher in den Operanden der binären Anweisung des Frequenzwechsels im Fall der Sequenzübertragung des genannten Magnetresonanzscanners eingibt.

2. Steuerungssystem nach Anspruch 1, bei dem die genannte grafische Umgebung eine Reihe von Instrumenten umfasst, die geeignet sind, die Programmierung der genannten Untersuchungen zu gestatten.

3. Steuerungssystem nach einem oder mehreren der vorangegangenen Ansprüche, das mit einer Client/Server-Architektur ausgestattet ist.

## Revendications

1. Système de commande pour un scanner à résonance magnétique capable de prendre en charge la réalisation des champs magnétiques produits par ledit scanner et l'enregistrement de données relatives aux signaux reçus de ce dernier et comprenant un processeur électronique incluant un logiciel de commande du scanner et un environnement graphique de développement tel à fournir les instruments de base pour le choix des examens et l'acquisition des images et **se caractérisant par** le fait d'inclure un micrologiciel DSP (8) contrôlant la mise à jour des valeurs de fréquence de transmission et les angles de la matrice de rotation des gradients en cas de séquences à couches multiples, ledit micrologiciel DSP (8) étant conçu de sorte qu'une séquence appropriée d'instruction amène ce même micrologiciel DSP (8), par la génération d'interruptions et de messages pendant l'exécution de la séquence même, à lire les nouvelles valeurs de fréquence de transmission et les matrices de rotation des tableaux spécifiques générées par le calculateur, et configurant directement les valeurs et angles dans la mémoire de séquence des operandi d'instruction binaire de changement de fréquence en cas de transmission de séquence dudit scanner à résonance magnétique.

2. Système de commande selon la revendication 1 dans ledit environnement graphique comprenant une série d'instruments propres à permettre la programmation desdits examens.

3. Système de commande selon une ou plusieurs des revendications précédentes doté d'une architecture de type Client / Serveur.
